# EUROPEAN PATENT APPLICATION

(11) **EP 2 143 451 A1**
(43) Date of publication of application: **13.01.2010**
(21) Application number: 08012552.9
(22) Date of filing: 11.07.2008
(51) Int. Cl.: A61L 27/36, A61L 27/54, A61L 31/16

(54) **Bone implant application**

(71) Applicant: Nobel Biocare Services AG, 8058 Zürich-Flughafen (CH)
(72) Inventor: Fäldt, Jenny, 431 31 Mölndal (SE)
(74) Representative: Byström, Kurt Linus

(57) **Abstract**

The invention concerns in combination, a bone implant, and one or more bioactive substances which promote osseointegration of said bone implant. At least one of said one or more bioactive substances may be capable of reducing proliferation, differentiation, and/or activity of osteoclasts, stimulating proliferation of osteoblasts, and/or reducing inflammation.

## Description

### Field of the invention

The present invention relates to a bone implant application, and more specifically to a combination of a bone implant and one or more bioactive substances which promote osseointegration of the bone implant, use of one or more bioactive substance(s) which is(are) capable of promoting osseointegration, and a method for a bone implant.

### Background of the invention

Implants and implant elements and methods for permanent anchoring prostheses such as dental replacements in mineralized tissue have been known for a long time. To prevent loosing the implant, it is important to establish direct contact (i.e. direct application) between the outer surfaces of the implant or implant element and the surrounding body bone and tissue. When direct application has indeed been established, so-called osseointegration can be obtained and the implant is positioned firmly.

The treatment of edentulous patients usually includes careful patient selection, a detailed surgical protocol, and careful planning of the prosthetic constructions, which are to be used. The most successful clinical results have been achieved after treatment of patients with tight, compact bone in which it is fairly easy to obtain primary as well as secondary stability of the implant. However, less successful results have been reported after treatment in bone of softer quality. The difficulties have manifested themselves in the break-up of a stable implant fixation, for example that primary stability is obtained but not secondary stability following the healing period. Various methods have been proposed to overcome these difficulties. These methods generally involve using additional new means for implant surfaces, for example coating with hydroxyapatite, or preparation of implant surface irregularities to improve the implant fixation.

It is recognised that, under certain conditions, implants made of commercially pure titanium or titanium alloys permit osseointegration with surrounding bone tissue so that intimate contact can be obtained between implant and tissue. Titanium implants anchored in bone can be used as fixtures for tooth replacements and tooth prostheses, or for other types of prostheses for example, but not limited to, finger joints, prosthetic eyes, prosthetic ears, hearing aids or devices for example for healing of broken bones.

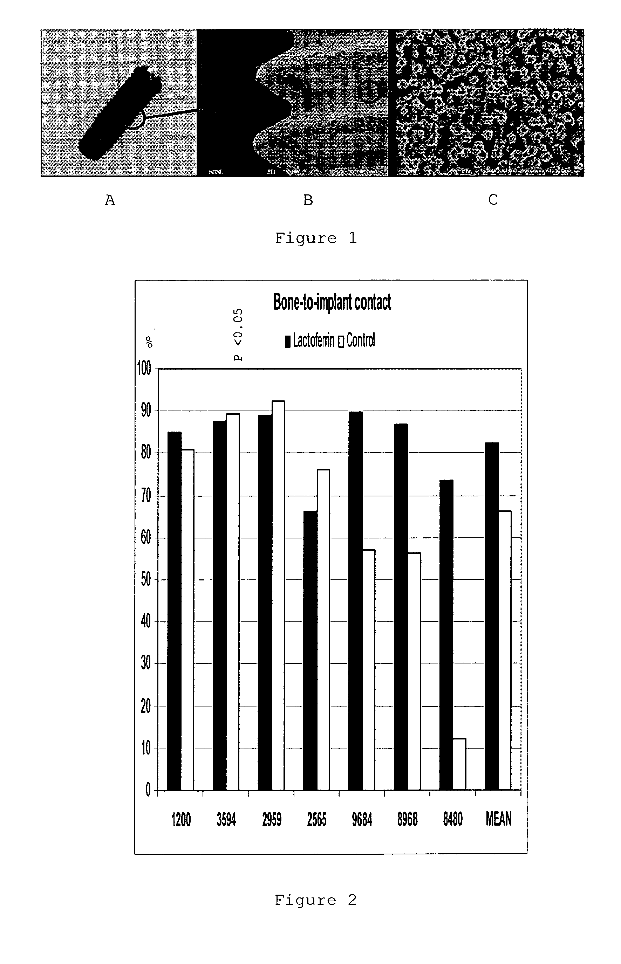

NobelBiocare provides a surface material TiUnite® (Swedish Patent 7902035-0) which has a unique porous surface structure suitable for different products and in particular bone implants, such as dental implants. TiUnite® is produced from a highly crystalline and phosphate enriched titanium oxide, which insures a microstructured topography on the surface of the implant characterized by the presence of uniformly distributed open pores in the low micrometer range, as shown in Figure 1.

It is already known to use various types of bioactive substances intended to improve the incorporation of implant in bone. For example the patent publications WO 95/33502, WO 96/40014 and WO 98/17330 suggest use of substances belonging to the superfamily TGF-β, for example so-called BMPs (Bone Morphogenetic Proteins) in order to initiate and stimulate bone growth. BMPs however, would not seem to provide the necessary signals for promoting enhanced osseointegration on their own.

WO 2004/010887 describes the use of growth-stimulating substances on, or in, the implant that results in new bone formation around the implant.

US5686116 discloses a method to enhance attachment, fixation and stabilization of a bone implant. The method comprises administering to a patient to receive the implant an amount of a pharmaceutically acceptable group IIIa element-containing compound sufficient to provide therapeutically effective levels of the element. The compound may be bound to a protein naturally present in the body, such as lactoferrin. However, the effect of the protein as such is not disclosed.

WO08043175 discloses a composition for inhibiting biofilm-embedded microorganisms, which comprises, i.a. an antimicrobial agent, such as lactoferrin, for use as a coating on an implant. However, WO08043175 does not disclose bone implants having a bone apposition surface for osseointegration with bone tissue. Furthermore, WO08043175 only discusses the anti-bacterial effects of lactoferrin.

Obtaining primary stability as well as secondary stability following a healing period, preferably without, or with little, bone resorption is a concern with bone implants.

Thus, there is a need for an improved bone implant and/or method for implanting a bone implant. Hence, an improved bone implant and/or method for implanting a bone implant would be advantageous. In particular a bone implant and/or method for implanting a bone implant allowing reliable anchoring in bone tissue; increased surgical flexibility; improved surgical control, e.g. by improved outcome of the medical procedure; and/or cost-effectiveness, e.g. by patient recovery time, potential side effects; etc. would be advantageous.

### Summary of the invention

Accordingly, embodiments of the present invention preferably seek to mitigate, alleviate or eliminate one or more deficiencies, disadvantages or issues in the art, such as the above-identified, singly or in any combination by providing a bone implant, a method of implanting a bone implant, and use of one or more bioactive substances for preparing a combination of a bone implant and the substance(s) according to the appended patent claims.

According to an aspect, a combination comprises a bone implant, and one or more bioactive substances which promote osseointegration of said bone implant.

At least one of said one or more bioactive substances may be capable of reducing proliferation, differentiation and/or activity of osteoclasts.

At least one of said one or more bioactive substances capable of reducing proliferation, differentiation and/or activity of osteoclasts may comprise ibandronate and/or lactoferrin.

At least one of said one or more bioactive substance(s) may be capable of reducing inflammation.

At least one of said one or more bioactive substances capable of reducing inflammation may comprise prarastatin and/or lactoferrin.

At least one of said one or more bioactive substances may be capable of stimulating proliferation of osteoblasts.

A at least one of said one or more bioactive substances capable of stimulating proliferation of osteoblasts may comprise at least one of lactoferrin, and a biologically functional derivative or fragment of lactoferrin.

The bone implant may be coated or impregnated on the surface with at least one of said one or more bioactive substances.

According to another aspect, use is made of one or more bioactive substance(s), which is/are capable of promoting osseointegration by inducing bone growth/formation and/or reducing inflammation for the preparation of the combination according embodiments of the combination.

According to another aspect, a method for a bone implant comprises preparing a site in bone of a patient for receiving a bone implant, local administration of one or more bioactive substances which promote osseointegration of said bone implant at the prepared site; and providing a bone implant in the prepared site.

The present invention provides for improved osseintegration of bone implants, in particular in connection with bone implants for dental structures such as dental prostheses. For example, the present invention provides for minimizing the time for osseointegration, which may include stimulation of bone growth in and around the implant in order to ensure secondary stability of implants for the benefit of the patient, who will experience a shorter time for secondary stability with less complications and a lower risk of loosing the implant. Another aspect, which the invention may provide for, is to minimize the time for obtaining secondary stability by decreasing the inflammatory responses following surgery. Furthermore, the combination according to the invention provide for local administration of the substance, whereby successful osseointegration of the implant may be facilitated.

It should be emphasized that the term "comprises/comprising" when used in this specification is taken to specify the presence of stated features, integers, steps or components but does not preclude the presence or addition of one or more other features, integers, steps, components or groups thereof.

### Figures

Figure 1 shows an scanning electron microscope (SEM) image of a bone implant with a TiUnite surface (A) and the surface of the implant at higher magnification (B) and the surface of the implant at even higher magnification depicting the microstructure surface pores with a diameter in the range of 1-10µm (C). These pores allow a better osseointegration, and in addition the pores allows for higher loading of the implant with anti-inflammatory compounds/drugs and osteoblast stimulating substances, such as lactoferrin, compared to regular machined implants.

Figure 2 shows a histogram of the bone-to implant-contact results of Lactoferrin dipped implants compared to the respective 0,9% NaCl dipped control implants in seven Göttingen minipigs measured by backscatter scanning electron microscope (B-SEM). The mean bone-to implant-contact value of Lactoferrin dipped implants compared to 0,9% NaCl dipped implant controls are depicted to the right.

### Detailed Description of Embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

Embodiments of the present invention provide means for improving osseointegration of implants, preferably implants in bone, such as the scull or jaw bone. It also minimizes the risk for loosing the implant due to uncontrollable bone resorption or lack of bone formation.

To understand the process of osseointegration the processes of bone formation has to be understood. Bone formation is characterized by a balance between bone resorption by osteoclasts and bone formation by osteoblasts. Implant-associated inflammation is often the result of manipulation of the bone structure in connection with the placement of the implant in the bone and characterized by inflammatory responses including infiltration of polymorphononuclear neutrophils (PMNs). These inflammatory responses are characterized, for example, by increased secretion of pro-inflammatory mediators such as cytokines (e. g. TNFα and IL-1), and toxic substances such as reactive oxygen species (ROS). These pro-inflammatory mediators seem to cause an imbalance in bone metabolism, by favouring bone resorption whereby bone loss is likely to occur.

Thus, prior to the initiation and stimulation of bone growth and as part of the recovery and wound healing after insertion of an implant, an inflammatory response often occurs in the tissue around the implant. The inflammatory response leads to an increase in the time of osseointegration, and thus secondary stability, and a prolonged inflammatory response may even lead to bone resorption and even implant failure. For patients who have been treated with implants and e.g. bone replacement materials, it is desirable to reduce the time of osseointegration to get improved osseointegration and to reduce the risk of loosing the implant.

The time for obtaining a stable osseointegration of an implant after insertion is composed of an "adaptation" process where the body reacts to the implant as to any other foreign invader by recruitment of inflammatory mediators and a "remodelling" process, where the implant, if accepted by the body, is incorporated into the bone by new bone formation around the implant, whereby secondary stability of an implant for subsequent mounting of prostheses is obtained. The two reactions exist in an equilibrium which is necessary to be pushed toward more bone formation than bone resorption in order to obtain osseointegration. The "remodelling" process can be speeded up and the "remodelling" time can be shortened as discussed above by stimulating the bone formation. A successful osseointegration however presupposes a short and successful adaptation process resulting in the reconstruction process to win the "battle". However, as it has been shown in the present invention, also in situations where there is little or no inflammation (and thereby little or no "adaption"), addition of bone growth signals are beneficial for the acceleration of osseointegration.

The "adaptation" period lasts until acceptance of the implant by the body is fully achieved. As discussed above, the adaptation starts with recruitment of neutrophils to the implant area. Both preparing a site for the implant, such as drilling of the hole in the bone, and providing the implant in the site, such as screwing in or otherwise placing the implant in the bone, will in most cases induce an inflammatory response as described above. The inflammatory response includes production and release by the neutrophils of ROS as well as the substances located in the granule such as e.g. the inflammatory mediators, lactoferrin, IL-1, IL-6, TNFα, etc., which among other things stimulate differentiation of osteoclasts and bone degradation. However, the release of such inflammation signalling substances seems to be necessary for initiation of the following "remodelling" state. If the immune system is not triggered by the insertion of the implant, the "adaption" period is never initiated which again may lead to no initiation of new bone formation and in the long run no "remodelling" or bone formation around the implant. Thus, the present invention sets out to secure and accelerate osseointegration of implants by stimulating bone growth or bone formation. Additionally or alternatively, the invention provides for decreasing inflammatory reactions around the implant, if present, following implantation and in this way diminishing the "adaption" period. Furthermore, the invention may provide for, if no inflammatory reaction occurs, supplying "adaption" signals, such as bone formation stimulating substance(s), or inflammatory signals, followed by acceleration of the bone formation around the implant.

Implant-associated inflammation is characterized by infiltration of immune cells including PMNs. These inflammatory responses are characterized, for example, by increased secretion of pro-inflammatory mediators such as cytokines (e. g. TNFα and IL-1, prostaglandins (e.g. PGD₂), thromboxanes (e.g. thromboxane B₄), leukotrines (e.g. leukotrine A₄) chemokines (e.g. IL-8) and toxic substances such as ROS. TNFα and IL-1 cause an imbalance in bone metabolism, by favouring bone resorption and TNFα blockers have an established role in the prevention of inflammatory bone loss in rheumatoid arthritis, a chronic inflammation of the joints. These immunological signals to the bone are supposed to be transmitted primarily via osteoblasts to induce osteoclast maturation, resulting in secondary osteoporosis. Chemokines induce cellular migration by binding to and activating the respective chemokine receptors. These receptors are expressed on the surface of different immune cell types allowing their specific attraction by the chemokines. Chemokines recruit PMNs to the site of the implant and these infiltrating immune cells can contribute to the already ongoing inflammatory processes. ROS include hydrogen peroxide (H₂O₂), hypochlorous acid (HC10), and free radicals such as the hydroxyl radical (·OH) and the superoxide anion (O₂⁻). The superoxide anion can react with nitrogen oxide (NO) and produce the highly reactive peroxynitrite oxidant. ROS are particularly unstable and will react rapidly and non-specifically with most biological molecules. ROS can damage cells by starting chemical chain reactions such as lipid peroxidation, or by oxidizing DNA or proteins. Damage to proteins causes enzyme inhibition, denaturation and protein degradation.

Neutrophils, are the most abundantly occurring white blood cell. The neutrophils belong to the first line of defence and migrate very rapidly from the blood vessel towards any kind of infiltrated pathogen or lesion. A lesion could for example be drilling a hole in the bone/jaw or placing an implant in a socket in the bone/jaw which triggers the infiltration of immune cells including the neutrophils towards the lesion and the implant. Normally the neutrophils phagocytise the pathogen or the intruder. The prey normally ends up in a vesicle, phagosome inside the neutrophils, with which the granules fuse to release their toxic substances. The neutrophils use two different mechanisms for killing the "intruder":
During the development and maturation of the neutrophils in the bone marrow, four different vesicles (granules) appear. Theses granules contain different substances as receptors used for migration and phagocytosis as well as toxic substances used for killing the invaders.

### a) Oxygen independent

The granules contain a large number of hydrolytic enzymes and anti-microbial polypeptides that are released into the phagosome resulting in, among other things, an acidification of the phagosome, hydrolyse of proteins by e.g. elastase and cathepsin G, degradation of peptidoglycan by lysozyme, and sequestration of essential nutrients as free iron by lactoferrin.

### b) Oxygen dependent

The neutrophils are able to produce and release ROS through the so-called NADPH-oxidase that assemblies in the phagosome membrane. Production of ROS e.g. superoxide anion, hydrogen peroxide, hypochlorous acid - will contribute to the killing of the "intruder".

However, sometimes the intruder is too large to be phagocytised which might lead to a so-called "frustrated phagocytosis". During a frustrated phagocytosis, the neutrophils release the ROS together with the toxic substances into the phagosome as usually, but this time the phagosome is not a closed system. Instead the ROS and toxic substances leak out in the surrounding tissue and thereby causing an inflammatory reaction with tissue damage as a consequence.

Some scenarios can be envisaged relating to the insertion of the implant and a resulting inflammatory response. The implant can e.g. be inserted in a fresh extraction socket immediately after a tooth has been extracted or inserted in the bone after drilling, e.g. the jawbone after drilling through the gums and jawbone. In both circumstances a vigorous implant-associated inflammatory response may be induced. In another case, e.g. if the implant is placed in an extraction socket, the socket is allowed to heal before the implant is inserted, which only triggers a minor or no inflammatory response. A minor or no inflammatory response may also occur when drill-and-implant- guided-surgery is used, such as according to the NobelGuide planning and treatment concept available via the applicant of the present invention. With a minor or no inflammatory response follows a minor initiation of the remodelling process, which seems to be required for osseointegration. Hence, triggering of the inflammatory response may be provided according to embodiments of the invention.

If part of the bone, for example a part or all of the jaw has been rebuild, e.g. after a head injury, it may often be needed to create the right signals for "remodelling" of the bone around an implant due to a lack of natural inflammation signals. Embodiments of the invention provide such signals in combination with the implant.

In this context bioactive substances are agents which promote or stimulate bone growth and/or bone formation. In this context bioactive substances also comprise agents which inhibit the pro-inflammatory responses, among those described above. Generally, any substance, endogenous or synthesized, which promote or stimulate bone growth/formation and/or inhibit pro-inflammatory responses, among those described above, can be applied according to embodiments of the invention, e.g. as a coating substance either solely or in any combination with other such compounds in or around the implant, or as in other forms in combination with the implant, such as by administration directly in an implantation site shortly before inserting the implant into the site.

One of the substances located in the granules of the neutrophils is lactoferrin. Lactoferrin is an 80 kDa iron-binding glycoprotein that belongs to the transferrin family of proteins. Beside its presence in neutrophils, lactoferrin is also found in other secretions e.g. tears, semen, saliva, and milk of most mammals. The serum levels in healthy subjects are around 2-7 µg/ml, but during an inflammatory process it can reach a concentration in the blood as high as 200 µg/ml.

In some embodiments, the bioactive substance comprises lactoferrin. Lactoferrin has been shown to have many different effects, such as anti-microbial activity by being an iron chelator, effects on cell growth and differentiation, embryonic development, myelopoiesis, endothelial cell adhesion, cytokine and chemokine production, regulation of the immune system by decreasing the release of osteolytic cytokines such as TNFα and IL-1β, modulation of the inflammatory response, gives wound closure in skin, reduce osteoclast activity by reducing osteoclastogenesis, anabolic factor to osteoblasts by increasing proliferation and differentiation, survival factor for osteoblasts by reducing apoptosis, increases protein matrix laid down and the mineralization during bone formation, anti-tumour effects. Lactoferrin receptors have been identified in many different cells, such as T cells, monocytes, liver cells, intestinal cells, and platelets. Lactoferrin maintains the osteogenic activity even when deglycosylated, in the holo and apo forms and in various small fragment of the molecule.

The effects on osteoblasts and bone formation by lactoferrin have been shown to occur at physiological concentrations, but also as low as 0.1µg/ml. When lactoferrin has been used in concentration as high as 1 - 9 g/day during 2 weeks on and 2 weeks off schedule in a Phase I trial against refractory solid tumours, no haematological, hepatic or renal toxicities were reported (Hayes TG., Falchook GF., et. al. Invest New Drugs 24(3); 2006). Hence, such high concentrations may be envisaged for the present invention.

The inhibition of osteoclast formation takes place at concentration >1 µg/ml. The N-lobe is more potent than the C-lobe or full-length recombinant human lactoferrin in inhibiting the development of osteoclasts. Other members within the Transferrin family might be interesting to test as well as the rh talactoferrin, which is a version of lactoferrin.

In a specific embodiment of the present invention, lactoferrin is used as the sole active substance. The advantage of using lactoferrin is that it is capable of inhibiting and/or reducing differentiation of osteoclasts as well as stimulating proliferation of osteoblasts. Also, lactoferrin is a substance capable of reducing inflammation. Embodiments of the present invention may also comprise the use of deglycosylated and active fragments or parts of lactoferrin.

In other embodiments, the stimulation of the bone formation is (further) supplemented with substances which inhibit the inflammatory processes. Such a substance may e.g. comprise lactoferrin, a biologically functional derivative or fragment of lactoferrin, and/or prarastatin.

Cornish et al. (Endocrinology 2004, 145(9), 4366-4374) demonstrated that lactoferrin increases bone formation *in vivo* by increasing osteoblast (bone formation) differentiation and reducing osteoblast apoptosis by up to 50-70 %, and further inhibits osteoclastogenesis. It is suggested that lactoferrin may have a physiological role in bone growth, and might have a utility as a local agent to promote bone repair.

Naot et al. (Clin. Med. Res. 2005, (3)2, 93-101) show that lactoferrin increases bone growth *in vivo* by injection over the right hemicalvarium in mice, and shows that this is due to inhibition of osteoclastogenesis and stimulation of the proliferation and differentiation of osteoblast-like cells.

Savarino et al. (J. Biomed. Material. Res. 2005, 75A, 324-332) describes that osteolysis (progressive periprosthetic bone loss) is responsible for approximately 70 % of implant failures. This is typically due to a particle-induced osteolysis at the implant-bone interface, mediated by the release of inflammatory mediators, leading to macrophage- and osteoclast-induced bone resorption and consequently implant loosening and implant failure. Savarino et. al. have used oral administration of anthraquinones in rats, and demonstrated increased bone volume. It is suggested that these drugs could have an effect of reducing osteoclast activity and suppress an inflammatory reaction. A systemic administration route is however unsuitable when a high local concentration is desired.

In embodiments, the applied substance(s) comprise, antibodies against and/or an inhibitor against the production and/or effect of any inflammatory intracellular or extracellular component/mediator involved in the pro-inflammatory events, e.g. antibodies against pro-inflammatory cytokines (e.g. anti-TNFα), inflammatory mediators (e.g. IL-8) and inhibitors against the receptors of pro-inflammatory cytokines (e.g. IL-1 receptor antagonist). Administration of anti-inflammatory cytokines, e.g. IL-10, may provide a mechanism by which particle-induced (or implant induced) inflammatory response can be modulated in vivo. Pre-treatment of macrophages with the anti-inflammatory cytokine IL-10 decreased the particle-induced cytokine release from the macrophage. Antibodies may be polyclonal or monoclonal and produced recombinantly or by any other known methods.

In other embodiments, the substance(s) provided in combination with the implant comprise at least one compound which inhibit the activity of any immune cells, inhibit their extravasations to the implant site (e.g. anti-selectins, anti-addressins, anti-integrins and anti-immunoglobulins), inhibit chemotaxis-related migratory responses of PMNs and other immune cells (e.g. anti-CC chemokines, anti-CXC chemokines, anti-C chemokines, anti-CX₃C chemokines) or combinations thereof.

In embodiments of the present invention, the substance(s) provided in combination with the implant comprises, but is not limited to, at least one compound which posses both osteogenic and anti-inflammatory properties e.g. lactoferrin, flavones (e.g. apigeni), anthraquinones (e.g. N,N'-Diethylamino-2,6-anthraquinone-disulfonamide and N,N'-(p-ethoxyphenyl)-2,6-anthraquinone-disulfon amide or combinations thereof.

In embodiments of the present invention, the substance(s) provided in combination with the implant comprises, but is not limited to, at least one compound with osteogenic properties, such as the BMPs e.g. transforming growth factor beta (TGF-β) isoforms (TGF-β1, TGF-β2 and TGF-β3) that signal through the TGF-beta type III receptor, adrenomedullin or inhibitors of bone resorption e.g. inhibitors of elastase, cathepsin G, lysozyme, and osteoclasts or combinations thereof.

In other embodiments of the present invention, the substance(s) provided in combination with the implant comprises, but is not limited to, at least one compound which belong to the non-steroidal anti-inflammatory drugs (NSAIDs): Salicylates such as Aspirin and Amoxiprin; Arylalkanoic acids such as Diclofenac and Indometacin; 2-Arylpropionic acids such as Ibuprofen Fenoprofen; N-Arylanthranilic acids such as Mefenamic acid; Pyrazolidine derivatives such as Phenylbutazone and Sulfinpyrazone; Oxicams such as Piroxicam and Meloxicam; COX-2 Inhibitors such as Celecoxib; Sulphonanilides such as Nimesulide; Others such as Licofelone and Omega-3 Fatty Acids.

In embodiments of the present invention, the substance(s) provided in combination with the implant comprises, but is not limited to, immunophilin ligands that are known as immunosuppressant drugs, i.e. FK506 (tacrolimus), cyclosporine A and rapamycin (sirolimus, rapamune) or combinations thereof.

In embodiments of the present invention, the substance(s) provided in combination with the implant comprises, but is not limited to, antioxidants e.g. glutathione, vitamin ascorbic acid (vitamin C), vitamin E (tocopherols and tocotrienols), melatonin, antioxidant enzymes such as catalase, superoxide dismutase, various peroxidases, and/or iron chelators such as transferrin, lactoferritin, ferritin, desferrioxamine, inducible oxide synthase (iNOS) inhibitors or combinations thereof.

In other embodiments of the present invention, the substance(s) provided in combination with the implant comprises, but is not limited to, agents which inhibits signalling molecules which are involved in the pro-inflammatory responses and/or bone resorption pathways, e.g. agents that suppress RANKL signalling, agents derived from plants which suppress osteoclasts cell signalling intermediates, e.g. curcumin (from turmeric), resveratrol (red grapes, cranberries and peanuts), tea polyphenols, genistein (soy), quercetin (onions), silymarin (artichoke), guggulsterone (guggul), boswellic acid (salai guggul) and withanolides (ashwagandha) Guggulsterone [4,17(20)-pregnadiene-3,16-dione] or combinations thereof.

In other embodiments of the present invention, the substance(s) provided in combination with the implant comprises, but is not limited to, agents which inhibit the production of arachidonic acid metabolites such as the COX-1 and COX-2 inhibitors, inhibitors of 5-lipoxygenaseor be different lipid mediators e.g. lipoxins, the new families of chemical mediators termed "resolvins" and "protectins" which like Lipoxin A4 have anti inflammatory effects.

In yet other embodiments of the present invention, the substance(s) provided in combination with the implant comprises, but is not limited to, heat shock proteins that are highly conserved proteins that protect cells against noxious or deleterious stimulus for example inflammation.

In the situation were an inflammatory response is not triggered and thereby the remodelling process required for osseointegration is lacking, lactoferrin or other osteogenic and/or osteoclast inhibiting agents, such as ibandronate, are applied in order to provide the right signals and trigger the bone formation signals.

Thus, in embodiments, the substance(s) provided in combination with the implant comprises, but is not limited to, any combinations of the agents mentioned above. Other ways of promoting bone growth and/or inhibiting pro-inflammatory responses would be obvious to the skilled person.

In one aspect of the present invention, bioactive substance(s) which stimulate(s) bone formation is/are coated or impregnated on the surface of the implant, e.g. as disclosed in WO 00/72777. In another aspect of the present invention, bioactive substance(s) which reduce(s) or inhibit(s) inflammation is/are coated or impregnated on the surface of the implant. The coating may be on a smooth surface or on a rough surface on the implant, for example by dipping an implant, such as an implant with a TiUnite® surface, in a suitable solution of the bioactive substance(s). The coating may be in the form of an even or uneven layer or in depots (spots). The substance(s) may also be incorporated in a matrix or other retaining materials on the surface of the implant suited for immediate or sustained release of the active substances. Microstructures in the implant can preferably be used as "caves" (Fig. 1B) for depots of the active substance(s). One such example of an implant with a microstructure is, e.g., implant with TiUnite® surface, coated with lactoferrin alone or in a slow release matrix.

In one aspect, the bone formation stimulation substance(s) is/are coated (e.g. sprayed) or impregnated on the surface of the implant in the form of a simple exposed layer or in a matrix or in depots. In another aspect, the anti-inflammatory substance(s) is/are coated (e.g. sprayed) or impregnated on the surface of the implant in the form of a simple exposed layer or in a matrix or in depots. The matrix or similar retaining material may be adapted for immediate or delayed/sustained release of the active substances. The different substances may alternatively be coated in different layers on the surface of the implant. The implant could be coated by dipping/incubation of the implant several times in a solution of the coating agent (with "drying" in-between). The surface of the implant could be coated with the active substance and covered by protection layer of, e.g. gelatine that is dissolved by the blood clotting after placement in the bone.

Microstructures (pores) in the implant are preferably filled with the active substance(s) during the coating process. These microstructures (Fig. 1B and C) allow a better osseointegration, and in addition allow for higher loading of the implant with bioactive substances, such as lactoferrin, compared to regular machined implants.

If more than one substance is applied, these may be applied together or consecutively, i.e. the substances are applied one after the other in accordance with the state of the osseointegration. Initially, anti-inflammatory effects are needed in order to suppress inflammatory reactions and degradation of tissue, or if such reactions are absent to initiate bone growth signals. In a later stage of the osseointegration, the bone/tissue growth is important and may therefore be stimulated by addition of suitable bone growth stimulating substances. Application of antibiotics, anti-swelling agents, painkillers and the like may also be contemplated for used according to the present application. Consecutive application of different (or the same) substance(s) require means for such application. Local injections may be contemplated. Delayed release depots may also be used, but the aim could also be achieved by the use of implants with mechanical means for stepwise application of the substance(s) to the implant when in place in the bone.

In another aspect, the bone formation stimulation substance(s) is/are provided in a cavity within the implant, e.g. as disclosed in WO 00/72778. In yet another aspect, the anti-inflammatory substance(s) is/are provided in a cavity within the implant. The substances may be deposited within the said cavity of the implant and allowed to diffuse via channels to the surface of the implant. The substance(s) may be incorporated in a matrix, a gel or other retaining material on the surface suited for immediate or sustained release.

In a further embodiment, the substance(s) may be applied in the cavity when the implant is in place in the bone as a gel, a solution or the like, or sucked into a sponge which is placed in the cavity of the implant.

In yet another embodiment, different substances can be applied successively to the cavity according to the need, for example an anti-inflammatory substance soaked into a sponge and placed in the cavity immediately after placement of the implant in a jaw bone; later (for example on day 2), a sponge soaked with a mixture of an anti-inflammatory substance and a bone growth stimulating substance is placed in the cavity instead of the previous sponge for a shorter or longer period of time and can even be replaced by a fresh soaked sponge as often as necessary and later (for example on day 7) a sponge soaked with bone growth stimulating substance(s) may be placed in the cavity for a shorter or longer period of time and can even be replaced by a fresh soaked sponge as often as necessary. Sponges with the same or different active substances may also be changed every day or more or less frequently if needed. Functionally alternatives to sponges may also be used.

In order for this construction to be useful, the above cavity containing implant should comprise a lid or other sealable means to secure the inside of the implant and the substance soaked means from interfering with chemical and/or mechanical substances from the outside of the implant, inside the mouth of the patient.

Any combination of the above-mentioned coating/impregnation and use of exchangeable soaked means may also be contemplated. For example, a cavity containing implant may be coated or impregnated on the outside of the implant and/or within the cavity with the first and optionally second bioactive substance, and in turn be supplemented with soaked means comprising second, third, etc., solutions.

In yet a further aspect, the implant may be coated with one active substance on the surface in a method as discussed above and provided with the same or another substance in a cavity of the implant with access to the surface as discussed above. In one example, an anti-inflammatory substance may be coated on the surface, and a bone growth stimulating substance may be applied to the cavity of the implant soaked in a sponge. Other combinations may be obvious to the skilled person.

The local application or administration of the bioactive substance(s), such as of inhibitors or anti-inflammatory substances, allows a much higher percentage of active substances to be used in the surroundings of the implant without being "diluted" in the entire body compared to a systemic application of the substances. The total amount used is thus lower than with systemic application for obtaining a desired concentration around the implant. Further, a higher local concentration may be acceptable, as the risk of intoxication of the organs or other vital part of the body is avoided. The higher local concentration may result in a faster down regulation of the inflammatory reactions and/or triggering the right signals for new bone formation. The local application or administration of bone growth or formation stimulating substance(s) may for the same reasons be preferred compared to systemic application. Further, most often bone growth is only desired locally, where a systemic application may lead to undesired bone growth in general. Treatment can occur over a prolonged period and can also be a combined treatment with different bioactive substances at different times in the treatment period.

Lactoferrin has been shown to stimulate osteoblast proliferation *in vitro* using concentrations as low as 0.1 µg/ml. Increasing the concentration to 1, 10 and 100 µg/ml decrease the osteoblast apoptosis, inhibit osteoclast development and stimulate osteoblast differentiation, respectively. These effects are present at concentrations of Lactoferrin that occur in vivo (1-100µg/ml) (Cornish et. al., Endocrinology. 2004 Sep;145(9):4366-74 and Caccavo D. et. al., Int J Clin Lab Res. 1999;29(1):30-5). Taking into account that the local concentration can be very high (Caccavo D. et. al, Int J Clin Lab Res. 1999;29(1):30-5), a suggested interval for local administration or coating of implants with Lactoferrin would be 0.1-5000 µg/ml. Local treatment with Lactoferrin could include administration using a scaffold or coating of an implant as well as applying a solution or gel directly at the site for implantation.

### Test for evaluation of the immune status of patient

In a further aspect of the present invention the immune status of the patient is checked in order to evaluate and choose the best combination of active substances. Different techniques might be useful for investigating the immune status. A common technique for collection of fluid is the use of filter paper strips. These are preferred due to easy access and transport and are used in e.g. enzyme assays, notably protease enzymes that lend themselves to specific colorimetric procedures.

Investigation of the appearance of connective tissue metabolites uses microcapillary tubes to prevent any risks of paper fibre contamination. The washing procedure disclosed by Skapi and Lehner (1976), Periodont Res 11, may be used. The skilled person would know alternatives.

As examples of potential marker sources in relation to microbial plaques may be mentioned: endotoxins (e.g. LPS), enzymes such as bacterial collagenase, bacterial hyaluronidase, metabolic end-products such as butyrate and proprionate from carbohydrates and lipids (correlates with gingivitis and periodontitis).

The positive correlation between the degree of inflammation, the volume of the gingival crevicular fluid (GCF) and H₂S generating potential of GCF-fluid may also be used to evaluate the patient.

Also, markers from the patient's cells can be used. The number of neutrophils in the gingival crevice is a useful marker of inflammation. Leucocytic enzymes e.g. alkaline phosphatase correlates with increasing inflammation and pocket depth, increased concentration of cathepsin D correlates with pocket depth. Lactoferrin showed a better correlation with clinical indices than neutrophils. Myeloperoxidase (MPO) gives a good correlation with an inflammatory response. In the patient's tissue the following markers can be detected: collagen, proteoglycans, matrix proteins, etc. Specific host factors can also be measured: immunoglobulins, immune response, complement, eicosanoids, cytokines (the levels of IL-6 and IL-10 in peri-implant disease group are positively correlated with clinical parameters), and the level of IL-10 might give information about the potency of an organism's integrated immune response for maintenance of inflammatory balance. The skilled person would know alternatives.

It's also possible to make a kit for the patient to determine the immune response (e.g. measurement of IL-10) after insertion of the implant in order to evaluate the best way to treat the pending implant-induced inflammation, and in essence be able to tailor a coating to suit a patient's specific needs.

In other embodiments, the bioactive substance comprises at least one of ibandronate and prarastatin. In some embodiments, the bioactive substance comprises ibandronate and prarastatin in combination. Ibandronate reduces or prohibits bone resorption, e.g. by influencing or reducing, such as reducing the proliferation, differentiation, and/or the activity of osteoclasts. Prarastatin is capable of reducing inflammation. Furthermore, statins has shown indications to stimulate bone formation. According to embodiments, either or both of ibandronate and prarastatin are combined with a bone implant according to the same principles as has been described herein with regard to other agents, such as lactoferrin, with the same positive effects on osseointegration.

Embodiments comprise a method for a bone implant. The method may comprise preparing a site in bone of a patient for receiving a bone implant, local administration or application of one or more bioactive substances which promote osseointegration of said bone implant at the prepared site, and providing a bone implant in the prepared site.

Local administration or application of the substance(s) may comprise local administration or application of at least one or more bioactive substances capable of reducing proliferation, differentiation and/or activity of osteoclasts. Local administration or application of the substance(s) may comprise local administration or application of at least one or more bioactive substances comprising ibandronate. Additionally or alternatively, the method may comprise local administration or application of at least one bioactive substance capable of stimulating proliferation of osteoblasts. Still additionally or alternatively, the method may comprise local administration or application of at least one bioactive substance capable of reducing inflammation. Still additionally or alternatively, the method may comprise local administrating or application of at least one bioactive substance comprising at least one of lactoferrin, a biologically functional derivative or fragment of lactoferrin, and prarastatin.

Preparing the site of the patient may comprise preparing an osteotomy, e.g. by drilling a hole into bone of the patient for inserting a bone implant. Additionally or alternatively, preparing the site may comprise using an osteotome to prepare the osteotomy or hole.

Local administration or application of at least one or more bioactive substances may comprise providing the bioactive substance on the surface of the implant, wherein the substance(s) is applied into the hole when inserting the implant. Alternatively or additionally, the substance(s) may be applied prior to inserting the implant, e.g. by painting, such as with a brush, or with a sponge or gel directly at the prepared site. Furthermore, the one or more substances and the implant may be provided in combination but distributed separately, such as in separate containers and/or vials. Before inserting of the implant into the prepared site, the implant is dipped into the substance, whereby the substance(s) is brought to the prepared site by means of the implant.

Providing the implant in the prepared site may comprise inserting the implant into the site, such as screwing the implant, such as a screw type implant, into the prepared site.

The bone implant may e.g. be a screw-type implant having one or more helical thread spirals or circular fins. The hole may e.g. be drilled into the jawbone, e.g. for applying a dental implant, the scull, e.g. for applying a maxillofacial implant, a backbone, e.g. for a spine implant, the hip bone, e.g. for a hip implant, etc.

A bone implant as used herein comprises an implant having at least one bone apposition surface devised for osseointegration with bone tissue, such as when inserted or at least partly embedded into a bone of a patient.

Some embodiments of the invention comprise use of one or more biocactive substances for providing a therapeutic treatment. The therapeutic treatment may comprise, e.g., osseointegration. The osseointegration may be osseointegration of a bone implant, such as a dental implant. The therapeutic treatment may additionally or alternatively comprise a prophylactic treatment of inflammatory deceases. The therapeutic treatment may additionally or alternatively comprise treatment of inflammatory deceases upon implantation of a bone implant. The one or more bioactive substances for the therapeutic treatment may comprise lactoferrin, prarastatin, and ibandronate, or any combination thereof.

Some embodiments of the invention comprise use of one or more biocactive substances for the manufacture of a medicament for providing osseointegration. The osseointegration may be osseointegration of a bone implant, such as a dental implant. Additionally or alternatively, the medicament may be a medicament for prophylactic and/or therapeutic treatment of inflammatory deceases. The manufacture may comprise providing the bioactive substance in combination with the bone implant.

### Example 1

### Effects of lactoferrin on osseointegration of titanium implants in Göttingen minipigs

In example 1 NobelReplace Tapered regular platform (RP) with TiUnite surface implants 4,3x13 mm was used. The site was prepared by drilling to protocol with 2 mm drill NP (narrow platform), dense bone drill RP, and screw tap RP. The implants and the drill protocols are available via the applicant of the present invention.

### Surgery

The 2^{nd} pre-molar tooth in the mandible on both sides were extracted in Göttingen minipigs and drilling was done in the distal or mesial alveolar using the protocol for placement of Replace tapered Rp 4,3x13 mm implants. A lactorferrin coated (coated by by incubation in 1000 µg/ml lactoferrin in 0,9 vol% NaCl) implant or a control implant (incubated in 0,9% NaCl) was placed in the fresh extraction socket after removing the roots. The implants were left 4 weeks for healing following surgery. Implants incubated in 0,9 % NaCl were used as a negative control and placed in the same way as the lactoferrin coated implants (although on separate sides of the jaw bone of the Göttingen minipigs to avoid any contamination). A cover screw and suture was used on the implant, if needed.

After four weeks following surgery, the implants were extracted together with a portion of the jaw on each side of the implant including one neighbouring tooth on each side of the implant. The samples were placed in 4 % PFA for fixation and left for at least one week. The specimens were evaluated by B-SEM (Back-scatter Scanning Electron Microscope). Evaluation using micro CT was also made. The results are summarized in table 1 below.

Simultaneously the TiUnite surface was evaluated with regard to the amount lactoferrin attached during dipping and with which rate the lactoferrin was released from the surfaces of the implant.

### B-SEM analysis

The jaws were cut longitudinally in the mesio-distal direction through the middle of the implants using an Exakt cutting unit (Exakt, Norderstedt, Germany) equipped with a diamond-coated band saw. The two resulting halves of the original specimen were embedded following complete dehydration in ascending grades of ethanol in a light-curing one-component resin (Technovit 7200 VLC, Kulzers). One half was used for evaluation with the B-SEM detector. The other half was used to produce ground sections for light microscopy (LM). For the B-SEM evaluation, the specimen was glued on an aluminium holder. The surface to be examined was highly polished with diamond pastes and thoroughly cleaned in an ultrasonic cleaner. Thereafter the polished surface was sputtered with a 6 nm thick carbon layer using an SCD-500 sputter coater (Bal-Tec, Liechtenstein). The specimen was examined with a Zeiss VPN-40 field cathode scanning electron microscope with the backscatter detector.

The resulting images were evaluated using Image Access (Imagic, Switzerland) software. The amount of the bone-to-implant contact was measured in percentage. The measurement was started from the first bone to implant contact at bone level around the body of the implant.

### Micro CT

The CT scanner used was a desktop Cone-Beam Micro CT Scanner with Microfocus X-Ray-source, 5 or 7 µm spot size, 30-70 kVp / 20-50 keV (160 µA), a 2048x256 elements, 24 µm pitch detector with a 6-72 µm nominal isotropic, 9 µm (10% MTF), Image matrix 512x512, 1024x1024 or 2048x2048 pixels resolution with a hp Integrity 64-bit 1 to 16 processor systems computer.

The amount of the bone-to-implant contact was measured in percentage.

### Results of Example 1

When 1000µg/ml lactoferrin was used in combination with a very gentle extraction (little trauma) the bone-to-implant contact was much higher compared with the control. It also seems to be more vascularisation around the lactoferrin coated implant, which is interesting as blood supply is very important.

To simulate the scenario were little or no inflammation and remodulation take place the surgery was carried out in two ways.

To induce a vigorous inflammatory response much trauma was simulated in the study by large problems with the extraction of the roots - i.e. the root was extracted in several pieces and with hard work. As seen in Table 1 and Fig. 2 the used lactoferrin concentration was not high enough to reduce the inflammatory reaction and bone loss. The first four sets of results in Table 1 simulate this situation.

Little trauma was also simulated in the study by a gentle and easy extraction of the roots - i.e. the roots come up in one pieces and without much work. Table 1 shows the results obtained after B-SEM and micro CT with regard to bone-to implant-contact in Lactoferrin dipped implants compared to the respective 0,9% NaCl dipped control implants in seven Göttingen minipigs. Higher numbers denote more bone-to implant-contact and more osseointegration. As seen in Table 1 and Fig. 2 the used lactoferrin concentration was enough to trigger the remodulation/new bone formation in the coated side compared with the control. The last three sets of results in Table 1 simulate this situation.

**Table 1: Quantification of bone-to-implant contact on both backscatter SEM and micro-CT evaluation.**

| **Specimen** | **back-scatter SEM %** | **micro CT %** | **Animal no.** |
|---|---|---|---|
| **Control** | 81.0 | 72.9 | 1200 |
| **Lactoferrin** | 84.8 | 75.3 | 1200 |
| **Lactoferrin** | 66.8 | 43.9 | 3594 |
| **Control** | 76.0 | 56.9 | 3594 |
| **Control** | 92.4 | 78.9 | 2959 |
| **Lactoferrin** | 89.0 | 78.4 | 2959 |
| **Lactoferrin** | 87.6 | 76.9 | 2565 |
| **Control** | 89.5 | 77.4 | 2565 |
| **Lactoferrin** | 86.6 | 82.3 | 9684 |
| **Control** | 56.3 | 77.6 | 9684 |
| **Control** | 57.0 | 72.9 | 8968 |
| **Lactoferrin** | 89.8 | 79.4 | 8968 |
| **Control** | 12.1 | 56.6 | 8480 |
| **Lactoferrin** | 73.5 | 65.9 | 8480 |

### Example 2

### Concentration of lactoferrin on titanium implants

Titanium plates (diameter 10 mm) with machine polished surfaces, titanium screw type implants with machine polished surfaces, and titanium screw type implants with TiUnite® surfaces were incubated for a minimum of 1 h up to at least 2h in lactoferrin (maximum 1000 µg/ml) dissolved in 0.9 % NaCl, and left to dry. The concentration of lactoferrin adsorbed to the implant was measured using different techniques. The measurement techniques used Techniques were XPS (X-ray Photoelectron Spectroscopy). DPI (Dual Polarisation Interferometer) is another possible measuring technique. The effect on the thickness of the coating layer can possibly also be evaluated using AFM (Atomic Force Microscopy).

### Example 3

In example 3, NobelReplace Tapered regular platform (RP) with TiUnite surface implants 4,3x13 mm was used. The site was prepared by drilling to protocol 2 mm drill NP (narrow platform), dense bone drill RP, and screw tap RP. 5 Göttingen minipigs were used. One coated implant and one control implant was placed per minimpig. The coated implants were coated with a combination of ibandronate (Ibandronate #I5784, Sigma-Aldrich) and pravastatin (Pravastatin #P4498, Sigma Aldrich). The implants were coated by incubation into a solution of 1000 µg/ml ibandronate and 400 µg/ml pravastatin. The control implants were incubated in a solution of 0.9% NaCl.

The implants and the drill protocols are available via the applicant of the present invention.

## Claims

1. In combination,
a bone implant, and
one or more bioactive substances which promote osseointegration of said bone implant.

2. The combination according to claim 1, wherein at least one of said one or more bioactive substances is capable of reducing proliferation, differentiation and/or activity of osteoclasts.

3. The combination according to any one of claims 1 or 2, wherein at least one of said one or more bioactive substances comprises ibandronate.

4. The combination according to claim 1, wherein at least one of said one or more bioactive substance(s) is capable of reducing inflammation.

5. The combination according to any one of claims 1 or 4, wherein at least one of said one or more bioactive substances comprises prarastatin.

6. The combination according to any one of claims 1 to 5, wherein at least one of said one or more bioactive substances is capable of stimulating proliferation of osteoblasts.

7. The combination according to any one of claims 1 to 6, wherein at least one of said one or more bioactive substances comprises at least one of lactoferrin, and a biologically functional derivative or fragment of lactoferrin.

8. The combination according to any one of claims 1 to 7, wherein said bone implant is coated or impregnated on the surface with at least one of said one or more bioactive substances.

9. The combination according to any one of claims 1 to 8, wherein at least one of said one or more bioactive substances is coated or impregnated on a surface of or deposited in a cavity in said bone implant, said cavity being in fluid contact with the outer surface of the bone implant through one or more channels.

10. The combination according to any one of claims 1 to 9, wherein at least one of said one or more bioactive substances is coated or impregnated on the surface of or deposited in a cavity in said bone implant in a matrix providing for immediate or delayed release of said substance(s).

11. The combination according to any one of claims 1 to 10, wherein the bone implant and the at least one of said one or more bioactive substances are provided as separate units, were at least one bioactive substance is either in dry or wet, e.g. in solution, form.

12. The combination according to any one of the previous claims, wherein one of said one or more bioactive substances include one or more drugs selected from the group comprising local acting antibiotic, painkillers and anti-swelling agents, etc.

13. The combination according to any one of the previous claims, wherein the bone implant comprises titanium, zirconium, alumina or ceramics.

14. The combination according to any one of the previous claims, wherein the surface of the bone implant comprises microstructures.

15. The combination according to any one of the previous claims, wherein the bone implant is a dental implant.

16. The combination according to any one of the previous claims, wherein one first bioactive substance is provided in a layer or depot for immediate release, which first substance is capable of reducing inflammation at the implantation site; and one second bioactive substance is provided in a second layer or depot providing for controlled release of said second substance which is capable of stimulating bone growth/formation.

17. Use of one or more bioactive substance(s) which is(are) capable of promoting osseointegration by inducing bone growth/formation and/or reducing inflammation for the preparation of the combination according to any of the claims 1 to 16.

18. A method for a bone implant, comprising
preparing a site in bone of a patient for receiving a bone implant;
locally administrating one or more bioactive substances which promote osseointegration of said bone implant at the prepared site; and
providing a bone implant in the prepared site.
